# EUROPEAN PATENT SPECIFICATION

(11) **EP 4 344 645 B1**
(45) Date of publication and mention of the grant of the patent: **12.08.2026**
(21) Application number: 23199769.3
(22) Date of filing: 26.09.2023
(51) Int. Cl.: A61B 6/04, A61B 6/10, A61B 6/42, A61B 6/50

(54) **PROTECTIVE COVER AND BIOPSY UNIT**
SCHUTZABDECKUNG UND BIOPSIEEINHEIT
COUVERCLE DE PROTECTION ET UNITÉ DE BIOPSIE

(30) Priority: 27.09.2022 JP 2022154076
(43) Date of publication of application: 03.04.2024
(73) Proprietor: FUJIFILM Corporation, Tokyo 106-8620 (JP)
(72) Inventor: TAJIMA, Takashi, Kanagawa (JP)
(74) Representative: Dehns Germany Partnerschaft mbB

(56) References cited:
- DE-A1- 102006 004 667
- DE-U1- 8 807 200
- US-A1- 2011 033 029

## Description

### BACKGROUND

### Technical Field

The present disclosure relates to a protective cover and a biopsy unit.

### Related Art

JP2008-237631A describes a mammography apparatus that captures a radiation image of a breast by disposing a radiation transmission body and a radiation impermeable body that constitute a grid to extend to be substantially parallel to a chest wall of the breast, and irradiating a solid-state detector with radiation output from a radiation source through the grid.

JP2016-515877A describes an X-ray imaging device in which an anti-scattering grid having a plurality of partition walls can be configured to be positioned with respect to the X-ray imaging device such that each partition wall of the plurality of partition walls extends along a direction substantially parallel to a coronal plane of a target during imaging of the target using the X-ray imaging device. The X-ray imaging device can operate in tomosynthesis mode for imaging a chest of the target, and can include the anti-scattering grid that is disposed between a chest platform and an X-ray detector.

In a case in which a biopsy for sampling tissue, such as a lesion, in the breast (that is, a biological tissue sampling examination, and hereinafter also referred to as a biopsy) is performed, there is a case in which the mammography apparatus is used to grasp a position of tissue as a sampling target in the breast. In such a case, the imaging of the breast and the sampling of the tissue are performed in a state in which a biopsy unit for the biopsy attached to the mammography apparatus. Since the biopsy unit has a puncture needle for sampling the biological tissue, in a case in which the biopsy unit is used, a protective cover that protects an imaging surface from the puncture needle is attached to an imaging table.

In the biopsy, in order to three-dimensionally grasp the position of the tissue in the breast, in some cases, stereo imaging or tomosynthesis imaging is performed in which a C-arm to which the radiation source is attached is rotated along an axis of rotation substantially parallel to a depth direction of the imaging surface, and the radiation is emitted from a plurality of positions having different irradiation angles with respect to the imaging surface. In a case of performing such imaging (hereinafter, referred to as biopsy pre-imaging), in some cases, a contrast of an image in which the breast is captured is decreased due to an influence of scattered rays of the radiation. In order to suppress the influence of the scattered rays, it is effective to use a scattered ray removal grid. However, in the scattered ray removal grid built in the imaging table as a standard, a direction of a boundary line between an absorption part that absorbs the radiation and a transmission part that transmits the radiation is not suitable for the biopsy pre-imaging, and vignetting of the radiation is increased in some cases.

DE 10 2006 004667 discloses a mammography method and device. DE 88 07 200 discloses an X-ray diagnostic device for mammography.

### SUMMARY

The technology of the present disclosure provides a protective cover and a biopsy unit capable of suppressing a decrease in a contrast of an image of a breast while suppressing vignetting of radiation in biopsy pre-imaging.

The present invention relates to a protective cover as claimed in claim 1.

In an embodiment, the scattered ray removal grid is disposed between the protective member and the imaging surface.

In an embodiment, the scattered ray removal grid is fixed to the protective member in a non-attachable/detachable form.

In another embodiment, the protective member is provided with a support shaft that supports the scattered ray removal grid, and the scattered ray removal grid is rotatable about the support shaft.

In an embodiment, the protective member has a rectangular shape, and the support shaft is disposed at any of four corners of the protective member.

In another embodiment, the protective member is provided with a guide part that guides the scattered ray removal grid to the facing position by engagement with the scattered ray removal grid.

In said embodiment, the guide part is able to accept the scattered ray removal grid from a side opposite to the chest wall side on which the chest wall of the subject is located.

In said embodiment, in a case in which a direction of the scattered ray removal grid along the first direction is defined as a depth direction of the protective member and a direction of the scattered ray removal grid along the second direction is defined as a width direction of the protective member, the guide part is a pair of guide rails that are provided on both ends in the width direction and extend in the depth direction.

In said embodiment, in a case in which a direction of the scattered ray removal grid along the first direction is defined as a depth direction of the protective member and a direction of the scattered ray removal grid along the second direction is defined as a width direction of the protective member, the guide part is able to accept the scattered ray removal grid from an end portion of the protective member in the width direction.

In said embodiment, the guide part is provided only on a side opposite to the chest wall side in the depth direction.

In an embodiment, a positioning part that regulates the scattered ray removal grid attached to the protective member at the facing position that faces the imaging surface is provided.

In an embodiment, the scattered ray removal grid is provided with a grip part.

In another embodiment, the scattered ray removal grid is provided on the protective member such that it can be displaced between the facing position that faces the imaging surface and a retreat position that retreats from the facing position, and the protective member includes a positioning part that regulates the scattered ray removal grid from being replaced at the facing position.

A further aspect according to the technology of the present invention relates to a biopsy unit that movably holds the puncture needle, the biopsy unit comprising the protective cover according to the invention, or any embodiment thereof.

The technology of the present invention can provide the protective cover and the biopsy unit capable of suppressing the decrease in the contrast of the image in which the breast is captured while suppressing vignetting of the radiation.

### BRIEF DESCRIPTION OF THE DRAWINGS

Fig. 1 is an exterior perspective view showing an example of a configuration of a mammography apparatus.
Fig. 2 is an exterior perspective view showing an example of a state in which a protective cover and a biopsy unit are attached to and detached from the mammography apparatus.
Fig. 3 is an exterior side view showing an example of the configuration of the mammography apparatus.
Fig. 4 is an exterior front view showing an example of the configuration of the mammography apparatus.
Fig. 5 is an exterior plan view showing an example of a state of a displacement of an external grid.
Fig. 6 is an exterior side view showing an example of a state in which radiation is emitted in the mammography apparatus.
Fig. 7 is a diagram summarizing a proper use of grids in the mammography apparatus.
Fig. 8 is an exterior side view showing an example of a configuration of a mammography apparatus as a comparative example.
Fig. 9 is an exterior front view showing an example of the configuration of the mammography apparatus as a comparative example.
Fig. 10 is an exterior plan view showing another example of the state of the displacement of the external grid.
Fig. 11 is an exterior plan view showing still another example of the state of the displacement of the external grid.
Fig. 12 is an exterior plan view showing still another example of the state of the displacement of the external grid.
Fig. 13 is an exterior plan view showing another example of the protective cover.

### DETAILED DESCRIPTION

Hereinafter, embodiments of the present disclosure will be described in detail with reference to the drawings.

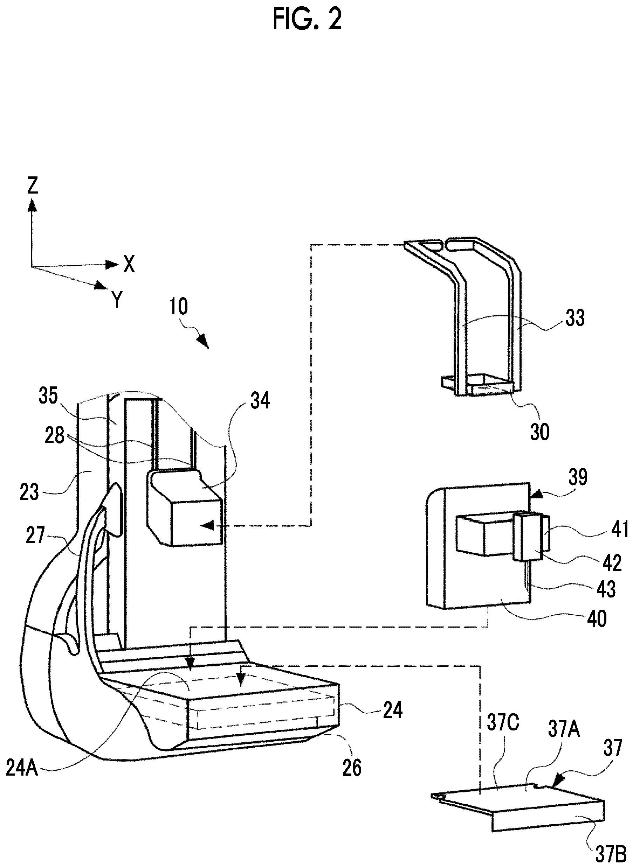

It should be noted that, in the following description, for convenience of description, a height direction, a width direction, and a front-rear direction (also referred to as a depth direction) of a mammography apparatus 10 are indicated by three arrows X, Y, and Z. First, the height direction is indicated by the arrow Z, an arrow Z direction indicated to by the arrow Z is an up direction of the mammography apparatus 10, and the opposite direction is a down direction. The height direction is a vertical direction. The width direction is indicated by the arrow X orthogonal to the arrow Z, a direction indicated by the arrow X is a right direction of the mammography apparatus 10, and the opposite direction is a left direction. The front-rear direction is indicated by the arrow Y as a direction orthogonal to the arrow Z and the arrow X, a direction indicated by the arrow Y is the front direction of the mammography apparatus 10, and the opposite is the rear direction. That is, in the mammography apparatus 10, a side of a stand 20 is the rear direction, and a side on which a subject A stands on the opposite side (see Fig. 2) is the front direction. In addition, in the following description, the expression using the side, such as an upper side, a lower side, a left side, a right side, a front side, and a rear side, has the same meaning as the expression using the direction.

In addition, in the present embodiment, "vertical direction" refers to the vertical direction in the sense of including an error generally allowed in the technical field to which the technology of the present disclosure belongs, that is, an error to the extent that it does not contradict the gist of the technology of the present disclosure, in addition to the exact vertical direction. In addition, similarly, "horizontal direction" refers to the horizontal direction in the sense of including an error generally allowed in the technical field to which the technology of the present disclosure belongs, that is, an error to the extent that it does not contradict the gist of the technology of the present disclosure, in addition to the exact horizontal direction.

In addition, in the present embodiment, "match" refers to the match in the sense of including an error generally allowed in the technical field to which the technology of the present disclosure belongs, that is, an error to the extent that it does not contradict the gist of the technology of the present disclosure, in addition to the exact match.

### First embodiment

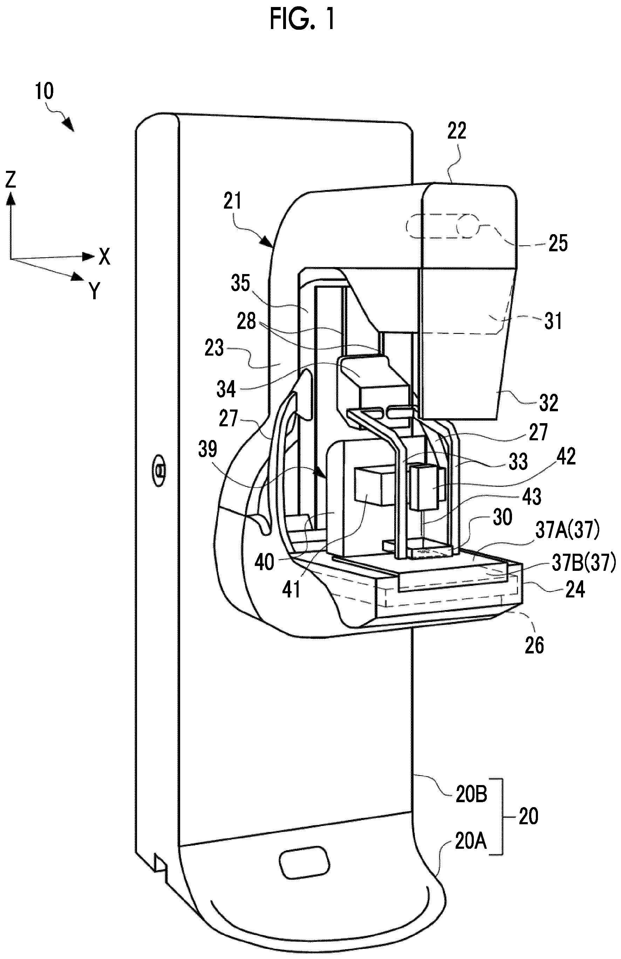

As shown in Fig. 1 and Fig. 2, the mammography apparatus 10 according to a first embodiment is a radiography apparatus that emits radiation to a breast M of a subject A as a subject and captures a radiation image of the breast M. The radiation is, for example, X-rays, but γ-rays may also be used. The subject A is located on a front side of the mammography apparatus 10. The mammography apparatus 10 is an example of a "mammography apparatus" according to the technology of the present disclosure.

The mammography apparatus 10 is connected to a console (not shown). The console has a function of acquiring the radiation image captured by the mammography apparatus 10 and displaying the acquired radiation image, in addition to a setting function of setting the mammography apparatus 10 according to an imaging order. The console is communicably connected to an image database server (not shown) via a network (not shown), such as a local area network (LAN).

The mammography apparatus 10 is provided with a stand 20 and an arm 21. The stand 20 is configured by using a seat 20A installed on a floor of a radiography room and a support column 20B that extends in the height direction from the seat 20A. The arm 21 has a substantially C-shape as viewed from the left side, and is connected to the support column 20B via a rotation axis. Since the arm 21 can be moved in the height direction with respect to the support column 20B, a height thereof can be adjusted according to a height of the subject A. In addition, the arm 21 can rotate about the rotation axis perpendicular to the support column 20B.

The arm 21 is configured by using a radiation source housing part 22, a body part 23, and an imaging table 24. A radiation source 25 is housed in the radiation source housing part 22. The radiation source housing part 22 has, for example, a housing structure having a longitudinal direction in the front-rear direction. The breast M of the subject A is placed on the imaging table 24. A radiation detector 26 is housed in the imaging table 24. The body part 23 integrally connects the radiation source housing part 22 and the imaging table 24. The body part 23 holds the radiation source housing part 22 and the imaging table 24 at positions that face each other. Handrails 27 for the subject A to grip are provided on both sides of the body part 23.

The breast M of the subject A is placed on the imaging table 24. The imaging table 24 comprises an imaging surface 24A on which the radiation transmitted through the breast M is incident. The radiation detector 26 is housed in the imaging table 24. The imaging table 24 is an example of an "imaging table" according to the technology of the present disclosure.

The radiation source 25 irradiates the breast M placed on the imaging table 24 with the radiation. The radiation emitted from the radiation source 25 is transmitted through a compression plate 30, and then incident on the breast M. The radiation detector 26 detects the radiation transmitted through the breast M, and outputs the radiation image. The radiation detector 26 is referred to as a flat panel detector (FPD). The radiation detector 26 may be an indirect conversion type that includes a scintillator converting the radiation into visible light and converts the visible light emitted from the scintillator into an electric signal, or may be a direct conversion type that directly converts the radiation into an electric signal.

An irradiation field limiter 31 is provided between the radiation source 25 and the imaging table 24. The irradiation field limiter 31 is also referred to as a collimator, and defines an irradiation field of the radiation to the imaging table 24.

A face guard 32 is attached to the radiation source housing part 22. The face guard 32 is made or coated with a material that does not transmit the radiation, and protects a face of the subject A from the radiation.

The compression plate 30 that interposes and compresses the breast M with the imaging table 24 is provided between the imaging table 24 and the irradiation field limiter 31. The compression plate 30 is made of a material that transmits the radiation. The compression plate 30 is disposed at a position that faces the imaging table 24. In the present embodiment, the compression plate 30 is formed in a box shape with an open upper surface side.

A moving mechanism 35 supports the compression plate 30 to be movable between the radiation source 25 and the imaging table 24. In addition, the movable part 34 is disposed between the compression plate 30 and the moving mechanism 35. The movable part 34 is held by a rail 28 provided on the moving mechanism 35 to be movable slidingly. The rail 28 stretches in the up-down direction.

The moving mechanism 35 includes, for example, a motor (not shown), a motor driver (not shown), and a feed screw mechanism (not shown). The motor rotates according to an electric drive signal output by the motor driver, and moves the compression plate 30 via the feed screw mechanism.

The compression plate 30 is attached to the movable part 34 via a pair of support arms 33. The movable part 34 is moved in the up-down direction together with the compression plate 30 by the moving mechanism 35. The up-down direction refers to, functionally, a direction in which the compression plate 30 is directed toward the imaging table 24 (that is, the down direction) and a direction in which the compression plate 30 is separated from the imaging table 24 (that is, the up direction). As described above, the compression plate 30 is configured to be movable in an aspect in which an interval with the imaging table 24 is changed.

A biopsy unit 39 is attached to the mammography apparatus 10. The biopsy unit 39 is a unit that samples tissue in the breast M to perform a biological tissue sampling examination. The biopsy unit 39 comprises a body part 40, an adjustment part 41, a needle holding part 42, and a puncture needle 43. The adjustment part 41 can be moved with respect to the body part 40, and as a result, an insertion position, an insertion angle, and an insertion amount of the puncture needle 43 with respect to the breast M are adjusted. The needle holding part 42 holds the puncture needle 43. The puncture needle 43 has, for example, a double structure of an outer needle and an inner needle. As an example, a procedure for sampling the tissue with the puncture needle 43 is as follows. In a state in which the puncture needle 43 is inserted into the breast M and a tip end portion of the puncture needle 43 reaches a position of the tissue that is a sampling target, the inner needle is protruded from the outer needle to sample the tissue into a tissue sampling region formed on the inner needle. After sampling the tissue in the inner needle, the inner needle is housed in the outer needle, and the puncture needle 43 is pulled out from the breast M. As a result, the tissue that is the sampling target in the breast M is sampled. In addition, the compression plate 30 is provided with an opening on a bottom surface, and the puncture needle 43 is inserted into the breast M via the opening. The puncture needle 43 is an example of a "puncture needle" according to the technology of the present disclosure.

In a case in which a biopsy is performed by using the biopsy unit 39, a protective cover 37 is placed on the imaging table 24. The protective cover 37 protects the imaging surface 24A from the puncture needle 43. That is, the contact of the puncture needle 43 with the imaging surface 24A is suppressed by the protective cover 37. The protective cover 37 comprises a flat plate-like protective member 37A and a front wall 37B bent from a front end of the protective member 37A. The breast M is placed on the protective member 37A. The protective member 37A is made of a material that can transmit the radiation (for example, an acrylic resin). A size of the protective member 37A is a size in a range that can support the breast M, and a plate thickness of the protective member 37A has a strength that can support the breast M. The protective member 37A is an example of a "protective member" according to the technology of the present disclosure.

As shown in Fig. 2, the biopsy unit 39 and the protective cover 37 are attached to the mammography apparatus 10 in a case in which the biopsy is performed. Stated another way, the biopsy unit 39 and the protective cover 37 are attachable to and detachable from the mammography apparatus 10. The protective cover 37 is attached to the imaging table 24 of the mammography apparatus 10 by utilizing, for example, magnetic attraction using a magnet. Specifically, the magnet is attached to the protective cover 37, and the magnet is magnetically attracted to a magnetizing plate provided on the imaging table 24 to attach the protective cover 37 to the imaging table 24. It should be noted that this is merely an example, and for example, the protective cover 37 may be attached to the imaging table 24 via a pressure-sensitive adhesive sheet, or the protective cover 37 may be attached to the imaging table 24 by fitting a part of the protective cover 37 into a recess provided in the imaging table 24.

The biopsy unit 39 is placed on the imaging table 24 after the protective cover 37 is attached to the imaging table 24. Specifically, the body part 40 of the biopsy unit 39 is placed above a rear end portion 37C of the protective cover 37. It should be noted that the biopsy unit 39 may be controlled to operate only in a case in which the protective cover 37 is attached to the imaging table 24.

Also, the compression plate 30 is a compression plate for the biopsy unit 39, and is attached to the mammography apparatus 10 by replacing the compression plate 30 with a compression plate for mammography (not shown) in a case in which the biopsy is not performed.

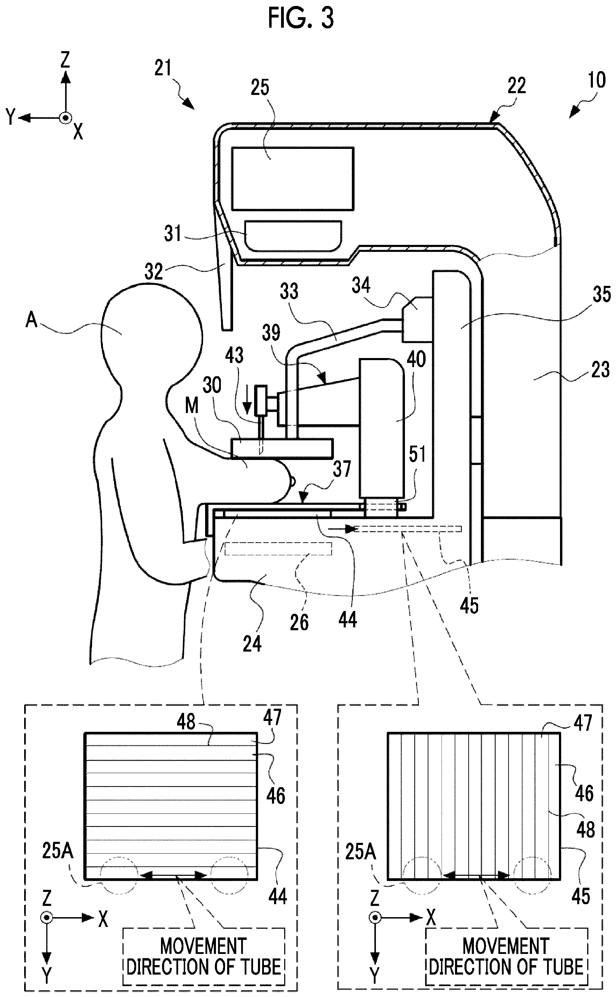

As shown in Fig. 3, for example, in a case in which the biopsy is performed, the puncture needle 43 is inserted into the breast M in a state in which the breast M is compressed by the compression plate 30. In such a case, it is required to accurately specify the position of the tissue (for example, a lesion tissue) that is the sampling target of the breast M. For this reason, as will be described below, in a case in which the biopsy is performed, in some cases, biopsy pre-imaging is performed to three-dimensionally specify the position of the tissue that is the sampling target in the breast M. In the present embodiment, stereo imaging is performed in which radiation R is emitted toward the breast from two left and right directions in which the irradiation angles with respect to the imaging table 24 are different.

Here, there is a case in which a scattered ray removal grid is built in the imaging table 24, as in a built-in grid 45. The scattered ray removal grid, as is well known, removes scattered rays generated by the radiation transmitting through the breast M. The built-in grid 45 is disposed inside the imaging table 24 on a side of the radiation source 25 with respect to the radiation detector 26. The built-in grid 45 is a flat plate-like member, and comprises a transmission part 47 that transmits the radiation R and an absorption part 46 that absorbs the radiation. The absorption parts 46 and the transmission parts 47 are alternately arranged, and a boundary line 48 between the absorption part 46 and the transmission part 47 is disposed inside the imaging table 24 in a state of extending in the front-rear direction (that is, a direction connecting a chest wall side and an anti-chest wall side of the subject A on the imaging surface 24A). Stated another way, the absorption parts 46 and the transmission parts 47 are alternately arranged in the left-right direction. Here, the front-rear direction is an example of a "first direction" according to the technology of the present disclosure.

Examples of a material of the absorption part 46 include a thin film of lead. In addition, examples of a material of the transmission part 47 include aluminum, paper, and carbon fiber.

In a case in which the stereo imaging is performed, in the mammography apparatus 10, the imaging is performed in a state in which the built-in grid 45 is moved to the anti-chest wall side (that is, a side of the body part 23) inside the imaging table 24. In the built-in grid 45, the boundary line 48 between the absorption part 46 and the transmission part 47 extends in a direction orthogonal to a movement direction of a tube 25A. Therefore, in a case in which the imaging is performed by moving the tube 25A by using the built-in grid 45, a central axis of a flux of the radiation R and the boundary line 48 are orthogonal to each other, and thus vignetting of the radiation is increased.

Therefore, in the stereo imaging, an external grid 44, which is a scattered ray removal grid integrated with the protective cover 37, is used instead of the built-in grid 45. The external grid 44 means a scattered ray removal grid disposed outside the imaging table 24, and is used here for distinction from the built-in grid 45 built in the imaging table 24. The external grid 44 comprises the absorption part 46 and the transmission part 47, similarly to the built-in grid 45. In the external grid 44, the absorption parts 46 and the transmission parts 47 are alternately arranged, and the boundary line 48 between the absorption parts 46 and the transmission parts 47 extends in the left-right direction. Stated another way, the absorption parts 46 and the transmission parts 47 are alternately arranged in the front-rear direction. As a result, in the external grid 44, the central axis of the flux of the radiation R and an extending direction of the boundary line 48 substantially match, so that the occurrence of vignetting of the radiation is suppressed in the stereo imaging. Here, the left-right direction is an example of a "second direction" according to the technology of the present disclosure. The absorption part 46 is an example of an "absorption part" according to the technology of the present disclosure, and the transmission part 47 is an example of a "transmission part" according to the technology of the present disclosure. The boundary line 48 is an example of a "boundary line" according to the technology of the present disclosure. The external grid 44 is an example of a "scattered ray removal grid" according to the technology of the present disclosure.

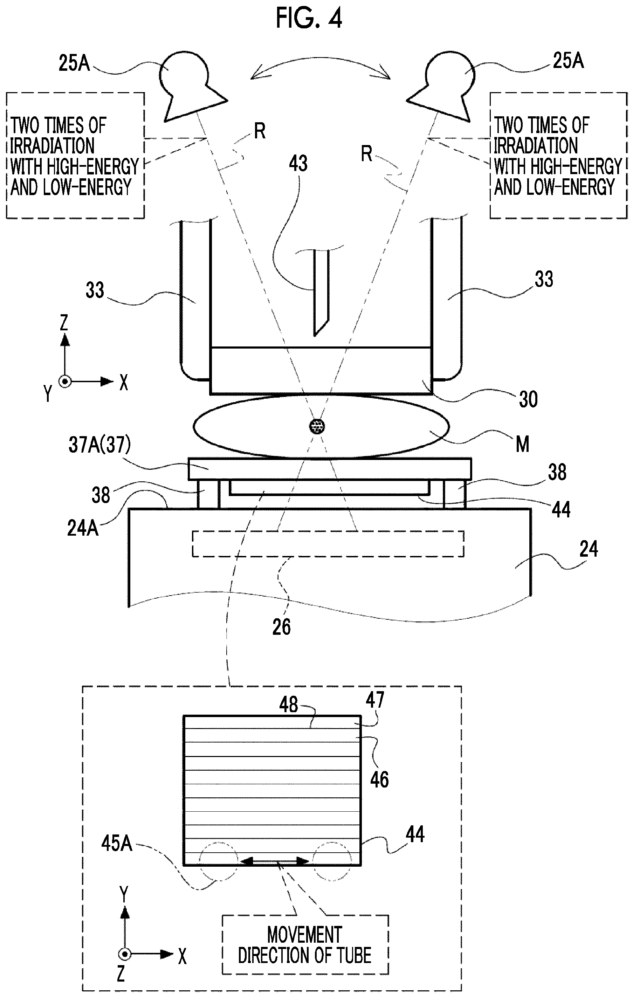

As shown in Fig. 4, the mammography apparatus 10 has a function of performing contrast imaging. As a contrast agent used for the contrast imaging, a contrast agent using iodine having a k-absorption edge of 33 keV (hereinafter, simply referred to as a "contrast agent") is generally used. The mammography apparatus 10 uses the breast M to which the contrast agent is administered as the subject, captures a low-energy image by using the radiation detector 26 by emitting the radiation R having a first energy lower than the k-absorption edge of the contrast agent, and captures a high-energy image by using the radiation detector 26 by emitting the radiation R having a second energy higher than the k-absorption edge of the contrast agent. Specific first energy and second energy are determined from specifications of the mammography apparatus 10, a desired image quality of the radiation image, and exposure of the subject or the like in addition to the k-absorption edge of the contrast agent, and are generally preferably 22 keV to 49 keV.

The contrast agent and the tissue, such as a mammary gland, have different absorption characteristics of the radiation R. Therefore, in the high-energy image captured as described above, a body tissue, such as the mammary gland or fat, is shown, and the contrast agent is clearly reflected. In addition, in the low-energy image, almost no contrast agent is shown, and the body tissue, such as the mammary gland, is clearly reflected. Therefore, a difference image showing a difference between the low-energy image and the high-energy image can be made to be an image in which a mammary gland structure is removed and the contrast agent is clearly shown. This imaging is referred to as contrast energy subtraction imaging.

Further, as described above, in a case of the biopsy, there is a case in which the stereo imaging is performed to three-dimensionally grasp the position of the tissue that is a biopsy target in the breast M. In the stereo imaging, an irradiation position of the radiation R is changed in the left-right direction, so that the irradiation angle of the radiation R is changed and the imaging is performed a plurality of times. The imaging with the radiation R having different energies (that is, the contrast energy subtraction imaging) is performed at each irradiation angle in the left-right direction in the stereo imaging. As a result, the tissue that is the biopsy target is clear in the image, and it is easier to grasp the three-dimensional position.

The stereo imaging is performed in a state in which the breast M is compressed between the compression plate 30 and the protective cover 37. The protective cover 37 includes a spacer 38, and a gap is formed between the imaging table 24 and the protective cover 37 by the spacer 38. The external grid 44 is provided between the protective cover 37 and the imaging surface 24A of the imaging table 24. The radiation R from which the scattered rays are removed by the external grid 44 is incident on the radiation detector 26. The high-energy image and the low-energy image can be obtained at each irradiation angle in the left-right direction. Then, after grasping the three-dimensional position of the tissue that is the biopsy target in the breast M by the stereo imaging, the puncture needle 43 is inserted into the breast M to sample the tissue that is the biopsy target.

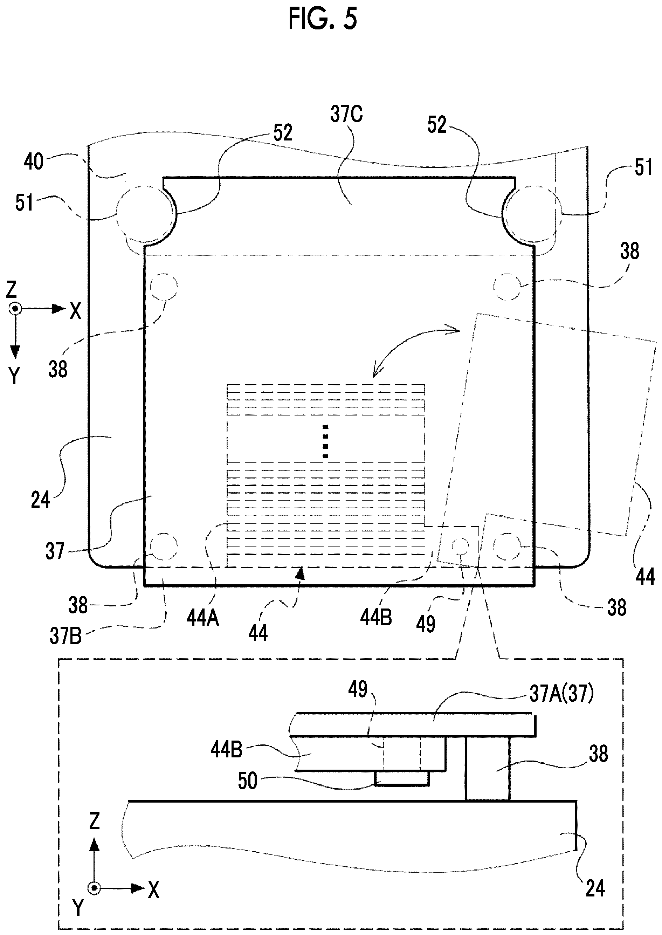

As shown in Fig. 5, in the protective member 37A of the protective cover 37, the rear end portion 37C is provided with an arc-like recess part 52. In addition, a pair of bases 51 are provided on the body part 40 of the biopsy unit 39. The base 51 has a columnar shape, and the recess part 52 has a shape corresponding to an outer periphery of the base 51. After the protective cover 37 is placed on the imaging table 24, the biopsy unit 39 is placed on the imaging table 24 from above. In such a case, the base 51 is placed at a position corresponding to the recess part 52. As a result, even in a case in which the protective cover 37 is moved in the front-rear direction or the left-right direction, the recess part 52 comes into contact with the base 51, so that a significant displacement of the position of the protective cover 37 is suppressed. It should be noted that a gap is provided between the outer periphery of the base 51 and an inner periphery of the recess part 52, and the movement of the protective cover 37 is allowed to some extent. As a result, the movement of the biopsy unit 39 with the movement of the protective cover 37 is suppressed, so that the displacement of the position of the puncture needle 43 provided in the biopsy unit 39 is suppressed.

The external grid 44 is integrated with the protective member 37A. It should be noted that, in the present embodiment, "integrated" is the meaning including a case in which the protective member 37A and the external grid 44 are separate members and integrated with each other by fixing the external grid 44 to the protective member 37A in a non-attachable/detachable form, and a case in which the protective member 37A and the external grid 44 are integrated with each other in an inseparable form by processing a surface of the external grid 44 or the like. The external grid 44 of the present example shown in Fig. 5 is the former case. In other words, the protective member 27A and the external grid 44 are separate members, and the protective member 27A and the external grid 44 are integrated with each other by fixing the external grid 44 to the protective member 37A in a non-attachable/detachable form.

Specifically, the external grid 44 is attached to the protective member 37A of the protective cover 37 via a support shaft 49. The support shaft 49 is provided on the protective member 37A and rotatably supports the external grid 44. Stated another way, the external grid 44 is rotatable about the support shaft 49. In the example shown in Fig. 5, the support shaft 49 is provided in a lower right corner in a case in which the rectangular protective member 37A is viewed in a plan view (that is, as viewed from the Z direction shown in Fig. 5). It should be noted that this is merely an example, and the support shaft 49 may be provided in an upper right corner, an upper left corner, or a lower left corner in a plan view of the protective member 37A. The support shaft 49 is an example of a "support shaft" according to the technology of the present disclosure.

In addition, the external grid 44 comprises a grid body 44A comprising the absorption part 46 and the transmission part 47, and an extending part 44B that extends from the grid body 44A toward the support shaft 49. The external grid 44 is rotatably attached to the support shaft 49 via the extending part 44B. One end of the support shaft 49 is attached to the protective member 37A, and the other end of the support shaft 49 is provided with a diameter expansion part 50. The extending part 44B is supported from below by the diameter expansion part 50. Here, the extending part 44B may be reinforced with a material having a strength higher than a strength of the grid body 44A. In addition, a metal plate may adhere to a surface of the extending part 44B to reinforce the extending part 44B. With such a configuration, the durability of the extending part 44B is improved against a load generated on the extending part 44B due to a rotation operation.

In this way, the external grid 44 can be displaced between a facing position that faces the imaging surface 24A and a retreat position that retreats from the facing position by rotating the external grid 44 about the support shaft 49. The external grid 44 is regulated from being displaced at the facing position. Stated another way, the external grid 44 is positioned at the facing position. In the example shown in Fig. 5, the external grid 44 rotated from the retreat position to the facing position is positioned at the facing position by coming into contact with the front wall 37B of the protective cover 37. The front wall 37B is an example of a "positioning part" according to the technology of the present disclosure.

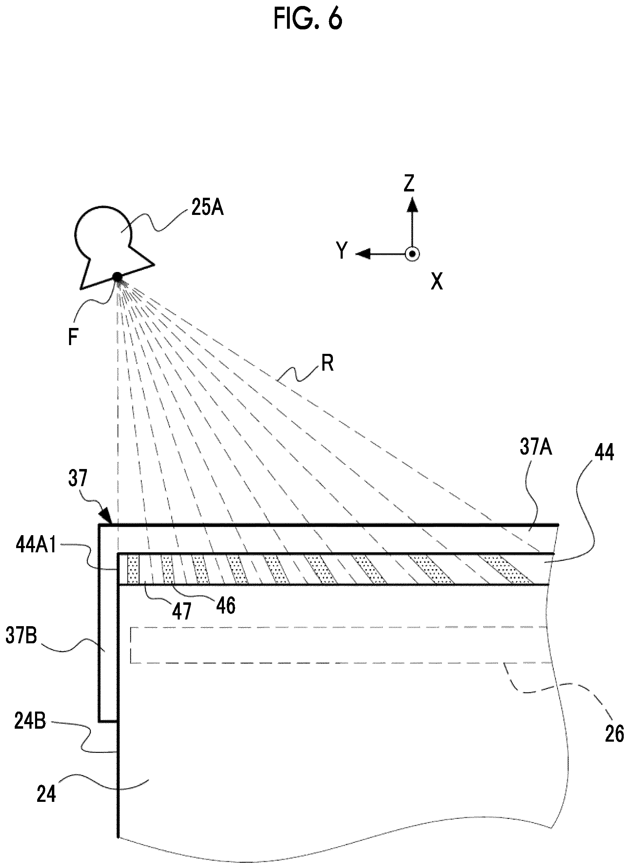

As shown in Fig. 6, in the facing position, the external grid 44 is located at a position closest to the chest wall side on the imaging table 24. For example, the external grid 44 is disposed at a position at which a front end surface 44A1 of the external grid 44 and a front end surface 24B of the imaging table 24 match. A focal position of the tube 25Ais disposed above the front end surface 24B of the imaging table 24. As a result, the image can be captured up to the chest wall side (that is, the front side) with respect to the breast M placed on the imaging table 24. Since the external grid 44 is located closest to the chest wall side of the imaging table 24, the scattered rays can be removed up to the chest wall side of the breast M that is an imaging target.

In addition, the external grid 44 is a so-called focused grid. That is, in the external grid 44, a plurality of boundary surfaces of the absorption parts 46 and the transmission parts 47 are not parallel to each other, and a surface obtained by extending each of the plurality of boundary surfaces is inclined in an aspect in which the surface passes through a focal position F of the tube 25A and is focused on one straight line parallel to the boundary surface. In the present example, since the focal position F of the tube 25A is disposed on the side of the front end surface 44A1 of the external grid 44, inclination angles of the plurality of boundary surfaces are gradually increased from the front end surface 44A1 toward the rear side. By setting the external grid 44 as such a focused grid, vignetting of the radiation R is further suppressed as compared with a parallel grid in which the plurality of boundary surfaces are parallel to each other.

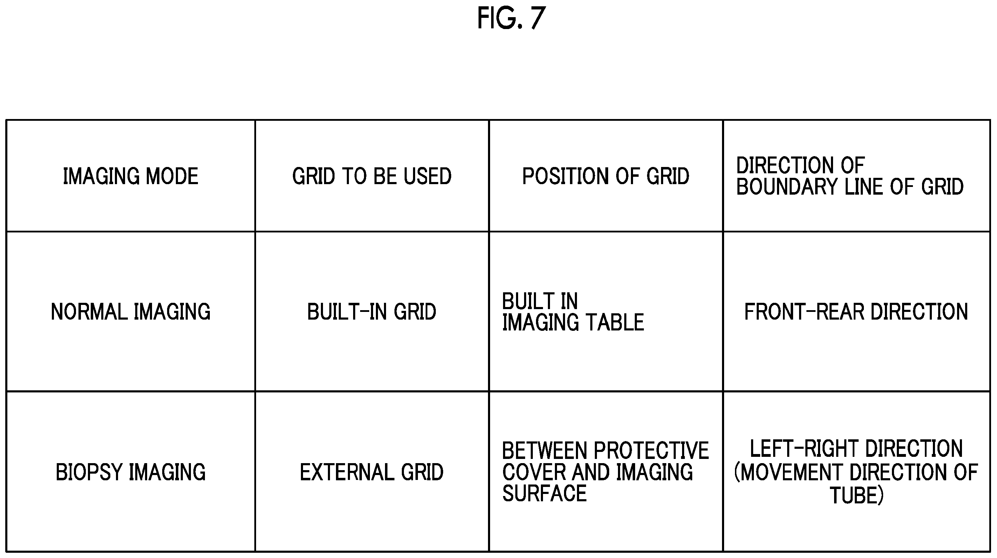

In Fig. 7, imaging modes and the grids used in the mammography apparatus 10 according to the present embodiment are summarized. As shown in Fig. 7, the built-in grid 45 is used in the imaging (that is, normal imaging) in a case in which the radiation source 25 is located at a position that faces the imaging table 24 in the vertical direction (that is, normal imaging). The built-in grid 45 is, as described above, built in the imaging table 24. In the built-in grid 45, the boundary line 48 between the absorption part 46 and the transmission part 47 extends in the front-rear direction.

On the other hand, in a case of the stereo imaging, the external grid 44 attached to the protective cover 37 is used. The external grid 44 is provided between the protective cover 37 and the imaging surface 24A. In the external grid 44, the boundary line 48 between the absorption part 46 and the transmission part 47 extends in the left-right direction (in the present example, the movement direction of the tube 25A).

As in the description so far, in a case in which the biopsy is performed in the mammography apparatus 10 according to the present embodiment, the protective cover 37 is used. The protective cover 37 comprises the external grid 44, which is an example of a scattered ray removal grid suitable for the stereo imaging, in addition to the protective member 37A.

That is, in a case in which the biopsy is performed, there is a case in which the stereo imaging is performed to three-dimensionally grasp the position of the tissue that is the biopsy target in the breast M. In the stereo imaging, the irradiation position of the radiation R is changed in the left-right direction, so that the irradiation angle of the radiation R is changed and the imaging is performed a plurality of times. With the present configuration, the external grid 44 can be disposed in a posture in which the extending direction in which the boundary line 48 of the absorption part 46 and the transmission part 47 of the external grid 44 is parallel to the left-right direction. For this reason, as compared with a case in which the external grid 44 is disposed in a posture in which the extending direction of the boundary line 48 is parallel to the front-rear direction, it is possible to suppress vignetting of the effective radiation R (radiation other than the scattered rays) that is obliquely incident from the radiation source 25 toward the imaging surface 24A. This is because, in a case in which the external grid 44 is disposed in a posture in which the extending direction of the boundary line 48 is parallel to the front-rear direction, the absorption part 46 that extends in parallel to the boundary line 48 and the central axis of the flux of the radiation R connecting the focal point of the tube 25A and the imaging surface 24A intersect with each other. That is, since the movement direction of the tube 25A and the extending direction of the absorption part 46 are orthogonal to each other, vignetting by the absorption part 46 occurs with respect to the radiation R emitted from the tube 25A.

Further, in a case of the biopsy, in order to acquire a breast image in which the biopsy target in which the new blood vessels are dense is emphasized, in some cases, the imaging in which the stereo imaging and the contrast energy subtraction imaging are combined is performed. The present configuration is particularly effective in such a case. This is because, in the contrast energy subtraction imaging, a difference between two images captured with the radiation having different energies is obtained, so that the contrast is likely to be decreased. In the present configuration, by using the external grid 44, the incidence of the scattered rays is suppressed, so that the decrease in the contrast can be suppressed. It should be noted that, as a method of suppressing the decrease in the contrast, it is also conceivable to increase a gain of a signal output by the radiation detector 26 in image correction processing. However, since the noise is also increased in a case in which the gain is increased, the method using the external grid 44 is preferable.

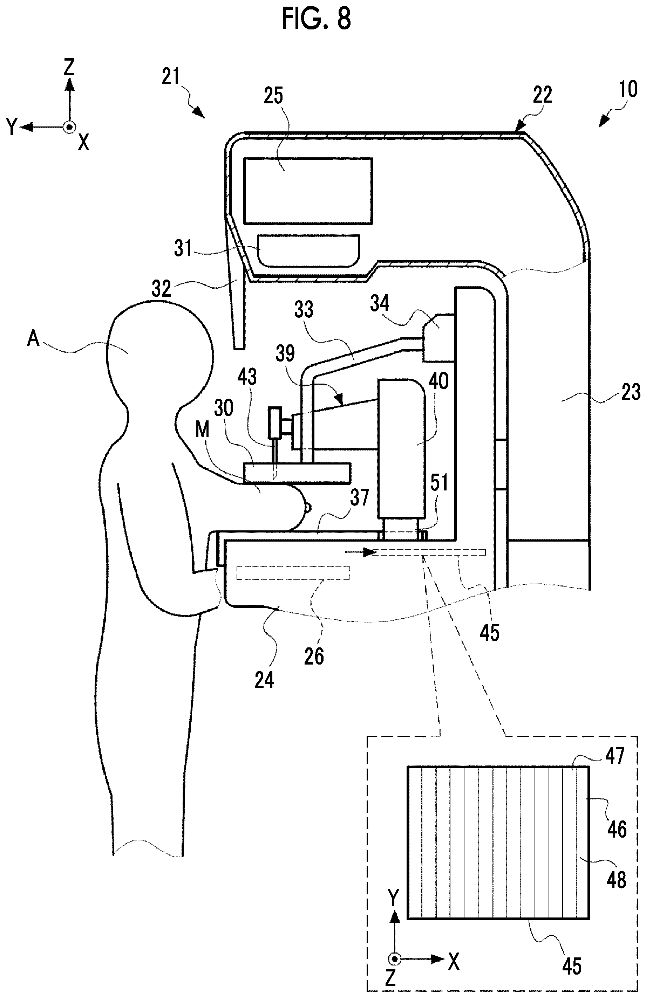

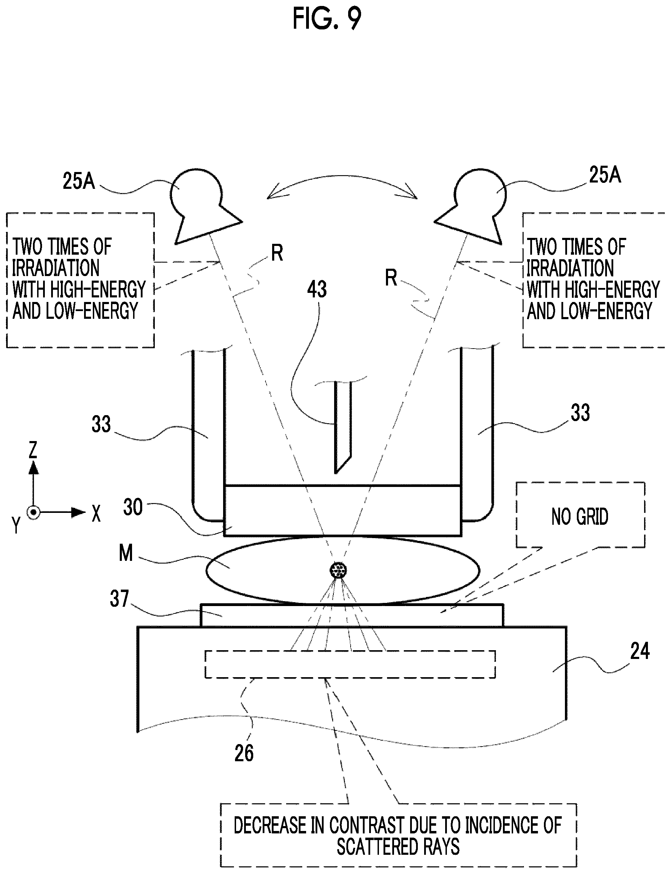

For example, as shown in Fig. 8 as a comparative example, a case will be considered in which the imaging in which the stereo imaging and the contrast energy subtraction imaging are combined is performed in a state in which the built-in grid 45 is made to retreat without using the external grid 44. In such a case, since the built-in grid 45 retreats, vignetting of the radiation R due to the orthogonality between the movement direction of the tube 25A and the extending direction of the boundary line 48 does not occur. On the other hand, as shown in Fig. 9, since the external grid 44 is not used, the scattered rays caused by the radiation R transmitted through the breast M are directly incident on the radiation detector 26. As a result, the contrast in the breast image is decreased. As described above, in the contrast energy subtraction imaging, since the difference between the two images captured with the radiation having different energies is obtained, the contrast is likely to be decreased, and thus the contrast in the breast image is further decreased. As a result, it is difficult to grasp the position of the tissue that is the biopsy target in the breast M. In the present configuration, by using the external grid 44, the incidence of the scattered rays is suppressed, so that the decrease in the contrast can be suppressed.

In addition, it is not assumed that the external grid 44 is provided in a protective cover for a normal biopsy according to the comparative example. For this reason, even in a case in which there is a grid to be externally attached to the imaging table 24, the imaging table 24 is already covered with the protective cover, and thus the grid cannot be disposed on the imaging table 24. In the protective cover 37 according to the present embodiment, since the external grid 44 is provided, it is possible to obtain the effect of removing the scattered rays by the external grid 44 while using the protective cover 37.

In the protective cover 37 according to the present embodiment, the external grid 44 is disposed between the protective member 37A and the imaging surface 24A. As a result, the external grid 44 and the imaging surface 24A are protected from the puncture needle 43 by the protective member 37A. In addition, for example, as compared with a case in which the external grid 44 is disposed on the opposite side (that is, an upper surface side) of the protective member 37A, an interval between the external grid 44 and the imaging surface 24Ais narrowed, so that the scattered rays can be effectively removed.

In addition, in the protective cover 37 according to the present embodiment, the external grid 44 is provided on the protective member 37A in an aspect that allows the displacement between the facing position that faces the imaging surface 24A and the retreat position that retreats from the facing position. Since the external grid 44 is displaced between the facing position and the retreat position, as compared with a case in which the external grid 44 is not displaced, the convenience of a user is improved such that the user can determine whether or not to dispose the external grid 44 at the facing position.

In addition, in the protective cover 37 according to the present embodiment, the protective member 37A is provided with the front wall 37B that regulates the displacement of the external grid 44 at the facing position. Since the external grid 44 is regulated at the facing position by the front wall 37B, the positioning accuracy of the external grid 44 is improved as compared with a case in which the external grid 44 is not regulated.

In addition, in the protective cover 37 according to the present embodiment, the protective member 37A is provided with the support shaft 49 that supports the external grid 44, and the external grid 44 is rotatable about the support shaft 49. Since the external grid 44 rotates about the support shaft 49, the external grid 44 is displaced to the facing position, and thus the work for the displacement is simplified.

In addition, in the protective cover 37 according to the present embodiment, the protective member 37A has a rectangular shape, and the support shaft 49 is disposed at the lower left corner of the protective member 37A. Since the support shaft 49 is disposed at the lower left corner, the influence on the radiation incident on the imaging surface 24A is small as compared with a case in which the support shaft 49 is disposed at the center of the protective member 37A.

In addition, in the protective cover 37 according to the present embodiment, the external grid 44 is integrated with the protective member 37A. Since the external grid 44 is integrated with the protective member 37A and the external grid 44 is disposed together with the protective cover 37, the disposition of the external grid 44 is simple as compared with a case in which the external grid 44 is a separate body from the protective cover 37.

Also, in the biopsy unit 39 according to the present embodiment, the puncture needle 43 is movably held, and the biopsy unit 39 comprises the protective cover 37. In a case in which the biopsy is performed by using the biopsy unit 39, the protective cover 37 is used. The protective cover 37 comprises, in addition to the protective member 37A, the external grid 44 suitable for the stereo imaging. In the present configuration, by using the external grid 44, the incidence of the scattered rays is suppressed, so that the decrease in the contrast can be suppressed.

### First modification example

In the first embodiment described above, a form example is described in which the external grid 44 is displaced between the facing position and the retreat position by rotating about the support shaft 49, but the technology of the present disclosure is not limited to this. In a first modification example, the external grid 44 slides in the front-rear direction with respect to the protective member 37A, so that the external grid 44 is displaced between the facing position and the retreat position.

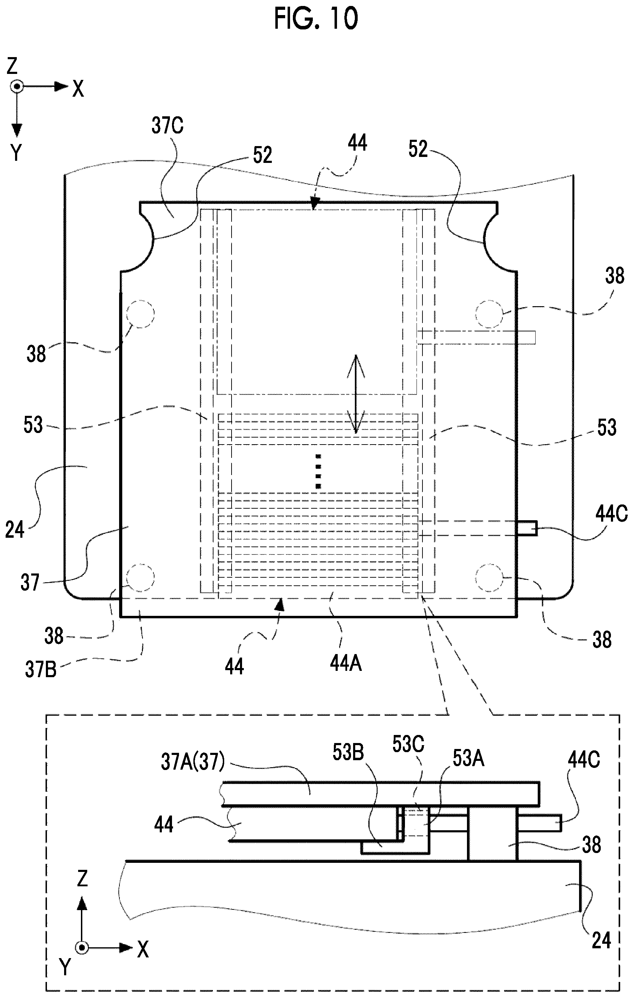

As shown in Fig. 10, the external grid 44 is attached to the protective member 37A via a pair of guide rails 53. The pair of guide rails 53 are rail-like members that extend in the front-rear direction. The pair of guide rails 53 are disposed in the left-right direction to be separated by a distance corresponding to a width of the external grid 44. Each of the pair of guide rails 53 has an L-shape in a cross section as viewed from the front-rear direction. Each of the pair of guide rails 53 comprises a side wall 53A that protrudes downward from the protective member 37A and a support part 53B that is bent from a distal end of the side wall 53A. The side wall 53A faces a side surface of the external grid 44 in the left-right direction, and regulates the movement of the external grid 44 in the left-right direction. The support part 53B supports a lower surface of the external grid 44 from below.

A long hole 53C is provided in the side wall 53A along the front-rear direction. A grip part 44C provided in the external grid 44 is inserted into the long hole 53C. The grip part 44C is a rod-like part that protrudes from the grid body 44A in the left-right direction. The external grid 44 is moved in the front-rear direction by the user gripping the grip part 44C and moving the grip part 44C in the front-rear direction. As a result, the external grid 44 is moved between the retreat position and the facing position. In such a case, the external grid 44 is guided by the pair of guide rails 53.

As in the description so far, in a case in which the biopsy is performed in the mammography apparatus 10 according to the first modification example, the protective cover 37 is used. The protective cover 37 comprises, in addition to the protective member 37A, the external grid 44 suitable for the stereo imaging. In the present configuration, by using the external grid 44, the incidence of the scattered rays is suppressed, so that the decrease in the contrast can be suppressed.

In addition, in the protective cover 37 according to the first modification example, the external grid 44 is integrated with the protective member 37A. Since the external grid 44 is integrated with the protective member 37A and the external grid 44 is disposed together with the protective cover 37, the disposition of the external grid 44 is simple as compared with a case in which the external grid 44 is a separate body from the protective cover 37.

### Second embodiment

In the first embodiment described above, a form example is described in which the external grid 44 is integrated with the protective cover 37, but the technology of the present disclosure is not limited to this. In a second embodiment, the external grid 44 is attachable to and detachable from the protective cover 37.

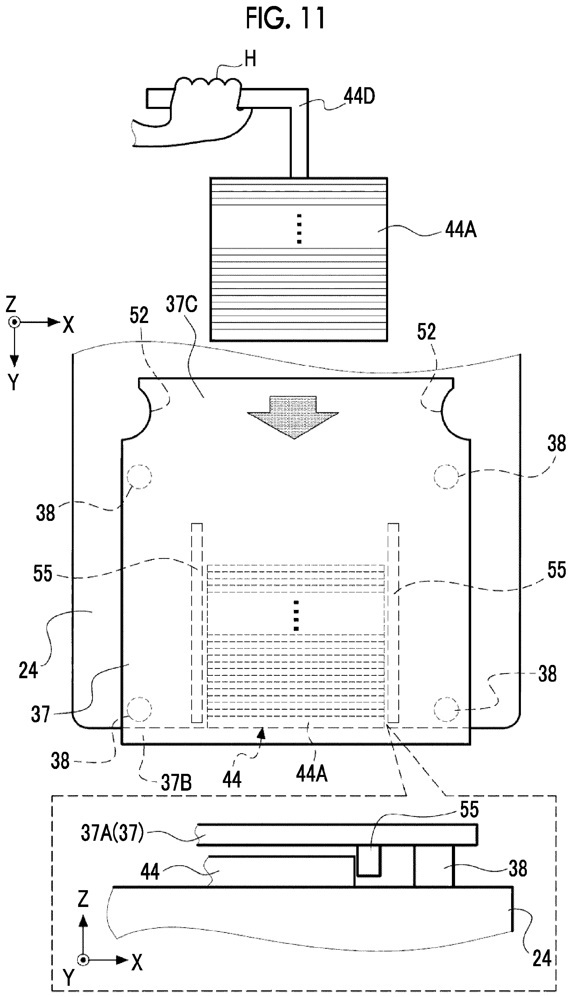

As shown in Fig. 11, a pair of guide rails 55 are provided on the protective member 37A of the protective cover 37. The pair of guide rails 55 are rail-like members provided on a lower surface of the protective member 37A. The pair of guide rails 55 extend along the front-rear direction and are disposed to be separated by a distance in the left-right direction according to the width of the external grid 44. The pair of guide rails 55 are provided at positions that face the side surfaces of the external grid 44. The pair of guide rails 55 is an example of a "guide part" and a "guide rail" according to the technology of the present disclosure.

The external grid 44 is provided with a grip part 44D. The grip part 44D is an L-like member in a plan view, and has one end attached to a rear end surface of the grid body 44A. In addition, the other end side of the grip part 44D can be gripped by a hand H of the user. As the grip part 44D is gripped by the user to move the external grid 44, the external grid 44 is inserted between the protective cover 37 and the imaging surface 24A from a side of the rear end portion 37C of the protective cover 37. In addition, the external grid 44 is inserted between the pair of guide rails 55. Stated another way, the pair of guide rails 55 can accept the external grid 44 from the rear side. Then, the external grid 44 is moved from the rear side to the front side of the protective cover 37 while being guided by the pair of guide rails 55. The external grid 44 is guided to the facing position by engaging the pair of guide rails 55 with the side surfaces of the external grid 44. In a case in which the external grid 44 comes into contact with the front wall 37B of the protective cover 37, the external grid 44 is positioned at the facing position. The grip part 44D is an example of a "grip part" according to the technology of the present disclosure. The front wall 37B is an example of the "positioning part" according to the technology of the present disclosure.

In addition, as the grip part 44D is gripped by the user to move the external grid 44, the external grid 44 is moved to the rear side from the facing position. Then, the external grid 44 is brought out from between the protective cover 37 and the imaging surface 24A. As described above, the external grid 44 is attachable to and detachable from the protective cover 37.

Before the external grid 44 is attached to the protective cover 37 or after the external grid 44 is detached from the protective cover 37, the external grid 44 may be directly handled by the hand H of the user. In such a case, the surface of the external grid 44 is reinforced. For example, the reinforcement may be performed by attaching a thin plate member made of a carbon fiber reinforced resin to the surface of the external grid 44.

As in the description so far, in the protective cover 37 according to the second embodiment, the external grid 44 is attachable to and detachable from the protective member 37A. Therefore, in a case in which the external grid 44 is not required (for example, in a case of imaging in which the tube 25A is not moved in the left-right direction), the protective cover 37 can be used even in a state in which the external grid 44 is detached.

In addition, in the protective cover 37 according to the second embodiment, the pair of guide rails 55 are provided on the protective member 37A. The pair of guide rails 55 guide the external grid 44 to the facing position by the engagement with the external grid 44. Since the guide rails 55 are provided, the external grid 44 is easily disposed at the facing position as compared with a case in which the guide rails 55 are not provided.

In addition, in the protective cover 37 according to the second embodiment, the pair of guide rails 55 can accept the external grid 44 from the rear side. Since the subject A is present on the front side of the mammography apparatus 10, the external grid 44 can be accepted from the rear side. Therefore, the external grid 44 is disposed at the facing position even in a case in which the subject A is present.

In addition, in the protective cover 37 according to the second embodiment, the external grid 44 is guided by the pair of guide rails 55 that extend in the front-rear direction of the protective member 37A. That is, since there is no member for guiding on the front side of the protective member 37A, the external grid 44 is inserted up to the front side of the protective member 37A. The chest wall of the subject A is present on the front side of the protective member 37A, and thus the external grid 44 is disposed up to the chest wall side.

In addition, in the protective cover 37 according to the second embodiment, in a case in which the external grid 44 comes into contact with the front wall 37B of the protective cover 37, the external grid 44 is positioned at the facing position. As a result, since the external grid 44 is regulated at the facing position, the positioning accuracy is improved as compared with a case in which the external grid 44 is not regulated.

In addition, in the protective cover 37 according to the second embodiment, since the grip part 44D is provided on the external grid 44, the operation for displacement of the external grid 44 is simplified.

It should be noted that, in the second embodiment described above, a form example is described in which the grip part 44D is attached to the external grid 44, but the technology of the present disclosure is not limited to this. An aspect may be adopted in which the grip part 44D is not provided on the external grid 44, and the external grid 44 is moved by pressing the external grid 44 from the rear side by a rod-like member that is a separate body from the external grid 44. In addition, an aspect may be adopted in which the external grid 44 is moved by pulling a string-like member attached to the front end surface 44A1 of the external grid 44 to the front side. In addition, the external grid 44 may have an aspect in which the user directly grips or moves the external grid 44 by the hand H.

### Second modification example

In the second embodiment described above, a form example is described in which the external grid 44 is inserted from the rear side of the protective cover 37, but the technology of the present disclosure is not limited to this. In a second modification example, the external grid 44 is inserted from the left-right direction (that is, the width direction) of the protective cover 37.

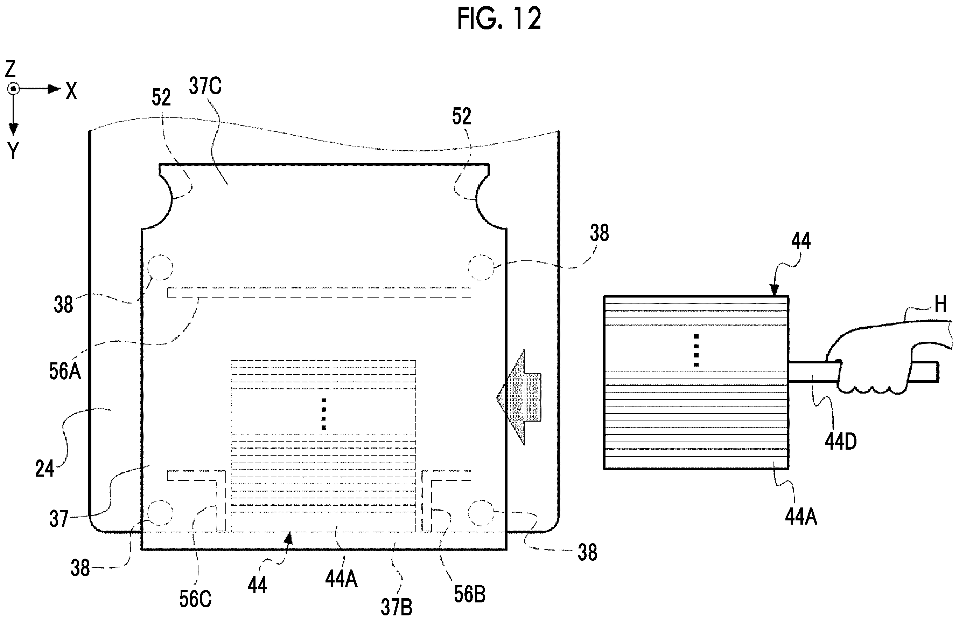

As shown in Fig. 12, a guide part 56 is provided on the protective member 37A of the protective cover 37. The guide part 56 comprises a first rail 56A, a second rail 56B, and a third rail 56C. The first rail 56A is a straight rail-like member that extends in the left-right direction. The second rail 56B and the third rail 56C are L-like rail-like members in a case in which the protective member 37A is viewed in a plan view. A distance between the first rail 56A and the second rail 56B is a distance corresponding to a distance in the front-rear direction of the external grid 44. Similarly, a distance between the first rail 56A and the third rail 56C is a distance corresponding to the distance in the front-rear direction of the external grid 44. The guide part 56 and the first rail 56A are examples of a "guide part" according to the technology of the present disclosure.

The first rail 56A and the second rail 56B make it possible for the external grid 44 to be accepted between the protective cover 37 and the imaging surface 24A from the right direction. In addition, the first rail 56A and the third rail 56C make it possible for the external grid 44 to be accepted from the left direction.

A distance between the second rail 56B and the third rail 56C is a distance corresponding to the distance in the left-right direction of the external grid 44. The movement of the external grid 44 in the front-rear direction is guided by the second rail 56B and the third rail 56C.

In the example shown in Fig. 12, as the grip part 44D is gripped by the user to move the external grid 44, the external grid 44 is inserted between the protective cover 37 and the imaging surface 24A from the right side of the protective cover 37. First, the external grid 44 is moved in the left direction while being guided by the first rail 56A and the second rail 56B. Further, the external grid 44 is moved in the front direction while being guided by the second rail 56B and the third rail 56C. Then, in a case in which the external grid 44 comes into contact with the front wall 37B of the protective cover 37, the external grid 44 is positioned at the facing position.

In addition, as the grip part 44D is gripped by the user to move the external grid 44, the external grid 44 is moved to the rear side from the facing position. Then, the external grid 44 is moved in the right direction. Thereafter, the external grid 44 is brought out from between the protective cover 37 and the imaging surface 24A. As described above, the external grid 44 is attachable to and detachable from the protective cover 37.

As in the description so far, in the protective cover 37 according to the second modification example, the guide part 56 can accept the external grid 44 from the left-right direction. Since the subject A is present on the front side of the mammography apparatus 10, the external grid 44 can be accepted from the left-right direction. Therefore, the external grid 44 is disposed at the facing position even in a case in which the subject A is present on the front side.

In addition, in the protective cover 37 according to the second modification example, the external grid 44 is guided by the first rail 56A, the second rail 56B, and the third rail 56C. In the protective cover 37, only the first rail 56A is provided in a movement range of the external grid 44 in the front-rear direction. That is, in the movement range of the external grid 44 in the front-rear direction, there is no member that regulates the movement of the external grid 44 in the front-rear direction on the front side of the protective member 37A, and thus the external grid 44 is inserted up to the front side of the protective member 37A. As a result, the chest wall of the subject A is present on the front side of the protective member 37A, and thus the external grid 44 is disposed up to the chest wall side.

### Third embodiment

In the first and second embodiments described above, a form example is described in which the external grid 44 can be moved relative to the protective cover 37, but the technology of the present disclosure is not limited to this. In a third embodiment, the external grid 44 is fixed to the protective cover 37.

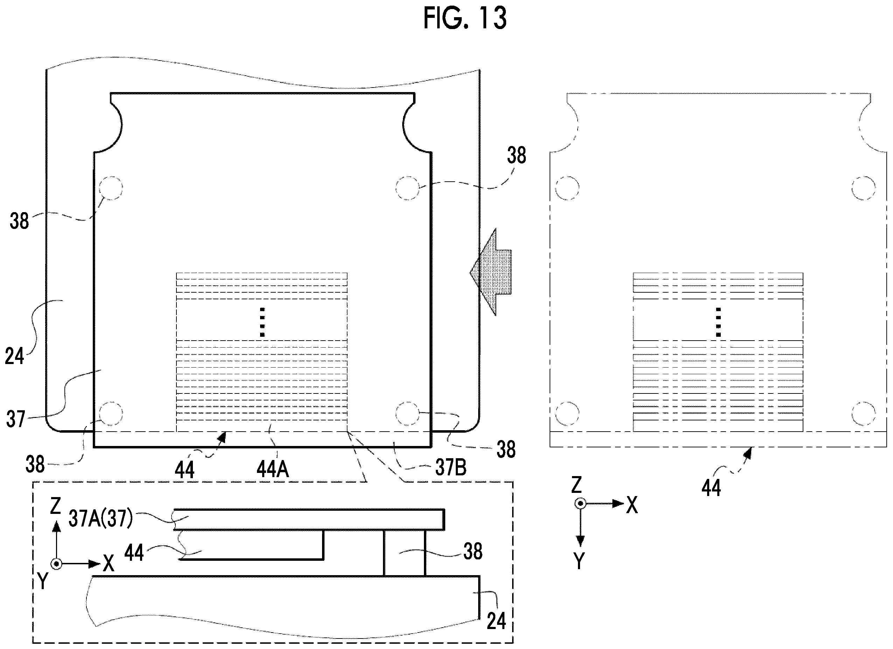

As shown in Fig. 13, the external grid 44 is fixed to the protective cover 37. The external grid 44 is fixed to the protective cover 37 by adhering to the protective cover 37 via a pressure sensitive adhesive, but this is merely an example. For example, the protective cover 37 and the external grid 44 may be fixed by a fastening member that fastens the protective cover 37, or the protective cover 37 and the external grid 44 may be fixed by being magnetically attracted by using the magnet.

A fixation position of the protective cover 37 with respect to the external grid 44 is adjusted in advance such that the external grid 44 is located at the facing position in a case in which the protective cover 37 is placed on the imaging table 24. The user also disposes the external grid 44 at the facing position by placing the protective cover 37 on the imaging table 24.

As in the description so far, in the protective cover 37 according to the third embodiment, the external grid 44 is integrated with the protective member 37A. Since the external grid 44 is integrated with the protective member 37A and the external grid 44 is disposed together with the protective cover 37, the disposition of the external grid 44 is simple as compared with a case in which the external grid 44 is a separate body from the protective cover 37.

It should be noted that, in the third embodiment described above, a form example is described in which the external grid 44 is fixed to the protective member 37A, but the technology of the present disclosure is not limited to this. For example, a form may be adopted in which the external grid 44 is embedded in the protective member 37A of the protective cover 37, or a form may be adopted in which a protective film is formed on the surface of the external grid 44 to function as the protective cover 37.

It should be noted that, in each of the embodiments described above, a form example is described in which the imaging in which the stereo imaging and the contrast energy subtraction imaging are combined is performed, but the technology of the present disclosure is not limited to this. For example, a form may be adopted in which only the stereo imaging is performed.

In addition, in each of the embodiments described above, a form example is described in which the stereo imaging is performed by moving the radiation source 25 in the left-right direction, but the technology of the present disclosure is not limited to this. For example, the radiation source 25 may be a so-called multi-radiation source that comprises a plurality of tubes 25A arranged along the left-right direction and emits the radiation R from each tube 25A. In the multi-source, the plurality of tubes 25A are arranged in the left-right direction, so that the stereo imaging can be performed, for example, by using the tubes 25A at both ends in the left-right direction.

In addition, in each of the embodiments described above, the stereo imaging in a case in which the biopsy is performed is described, but the technology of the present disclosure is not limited to this. For example, so-called tomosynthesis imaging, in which the breast M is imaged from a plurality of irradiation positions having different irradiation angles to obtain a tomographic image of the breast M, may be performed as the biopsy pre-imaging. Projection images of the breast M from the plurality of irradiation positions obtained by the tomosynthesis imaging are subjected to image reconstruction processing and used for generating the tomographic image of the breast M.

In addition, in each of the embodiments described above, a form example is described in which the biopsy unit 39 and the protective cover 37 are separate bodies, but the technology of the present disclosure is not limited to this. For example, the protective cover 37 may be fixed to the biopsy unit 39.

The described contents and the shown contents are the detailed description of the parts according to the technology of the present disclosure, and are merely examples of the technology of the present disclosure. For example, the description of the configuration, the function, the action, and the effect are the description of examples of the configuration, the function, the action, and the effect of the parts according to the technology of the present disclosure. Accordingly, it is needless to say that unnecessary parts may be deleted, new elements may be added, or replacements may be made with respect to the described contents and the shown contents within a range that does not deviate from the scope of the invention as defined by the claims. In addition, in order to avoid complications and facilitate understanding of the parts according to the technology of the present disclosure, the description of common technical knowledge or the like, which does not particularly require the description for enabling the implementation of the technology of the present disclosure, is omitted in the described contents and the shown contents.

## Claims

1. A protective cover (37) comprising:
a flat plate-like protective member (37A) that is provided to be attachable to and detachable from a mammography apparatus (10) including an imaging table (24) on which a breast of a subject is placed, wherein the imaging table (24) has an imaging surface (24A) on which radiation transmitted through the breast is incident, and wherein the protective member (37A) is configured to protect the imaging surface (24A) from a puncture needle (43) for sampling tissue from the breast; and
a scattered ray removal grid (44) that removes scattered rays generated by the radiation transmitting through the breast,
wherein the scattered ray removal grid (44) is a grid in which a plurality of transmission parts (47) that transmit the radiation and a plurality of absorption parts (46) that absorb the radiation are alternately arranged and a boundary line (48) between the transmission part (47) and the absorption part (46) extends in one direction,
**characterized in that**: the scattered ray removal grid (44) is integrated with or removably attached to the protective member (37A), and when the protective member is attached to the imaging table such that the scattered ray removal grid is disposed at a facing position that faces the imaging surface (24A), the scattered removal grid (44) is in a posture in which a direction in which the boundary line (48) extends is orthogonal to the direction connecting a chest wall side, on which a chest wall of the subject is located, and a side opposite to the chest wall.

2. The protective cover (37) according to claim 1,
wherein the scattered ray removal grid (44) is disposed between the protective member and (37A) the imaging surface (24A).

3. The protective cover according to claim 1 or 2, wherein the scattered ray removal grid (44) is fixed to the protective member (37A) in a non-attachable/detachable form.

4. The protective cover (37) according to any one of claims 1 to 3,
wherein the protective member (37A) is provided with a support shaft (49) that supports the scattered ray removal grid (44), and
the scattered ray removal grid (44) is rotatable about the support shaft (49).

5. The protective cover (37) according to claim 4, wherein the protective member (37A) has a rectangular shape, and the support shaft (49) is disposed at any of four corners of the protective member (37A).

6. The protective cover (37) according to claim 1, wherein the protective member (37A) is provided with a guide part (55) that guides the scattered ray removal grid (44) to the facing position by engagement with the scattered ray removal grid (44).

7. The protective cover (37) according to claim 6,
wherein the guide part (55) is able to accept the scattered ray removal grid (44) from a side opposite to the chest wall side on which the chest wall of the subject is located.

8. The protective cover (37) according to claim 7,
wherein, in a case in which a direction of the scattered ray removal grid (44) along the first direction is defined as a depth direction of the protective member (37A) and a direction of the scattered ray removal grid (44) along the second direction is defined as a width direction of the protective member (37A),
the guide part (55) is a pair of guide rails that are provided on both ends in the width direction and extend in the depth direction.

9. The protective cover (37) according to claim 6,
wherein, in a case in which a direction of the scattered ray removal grid (44) along the first direction is defined as a depth direction of the protective member (37A) and a direction of the scattered ray removal grid (44) along the second direction is defined as a width direction of the protective member (37A),
the guide part (55) is able to accept the scattered ray removal grid (44) from an end portion of the protective member (37A) in the width direction.

10. The protective cover (37) according to claim 9,
wherein the guide part (55) is provided only on a side opposite to the chest wall side in the depth direction.

11. The protective cover (37) according to any one of claims 5 to 10,
wherein a positioning part (37B) is provided, that regulates the scattered ray removal grid (44) attached to the protective member (37A) at the facing position that faces the imaging surface (24A).

12. The protective cover (37) according to any one of claims 5 to 11,
wherein the scattered ray removal grid (44) is provided with a grip part (44C).

13. The protective cover (37) according to claim 1,
wherein the scattered ray removal grid (44) is provided on the protective member (37A) such that it can be displaced between the facing position that faces the imaging surface (24A) and a retreat position that retreats from the facing position; and
wherein the protective member (37A) includes a positioning part (37B) that regulates the scattered ray removal grid (44) from being replaced at the facing position.

14. A biopsy unit (39) comprising:
the protective cover (37) according to any one of claims 1 to 13, the biopsy unit (39) movably holding the puncture needle (43).

## Patentansprüche

1. Schutzabdeckung (37), umfassend:
ein flaches plattenartiges Schutzelement (37A), das so vorgesehen ist, dass es an einer Mammographievorrichtung (10), die einen Bildgebungstisch (24) enthält, auf dem eine Brust einer Untersuchungsperson platziert wird, anbringbar und von dieser abnehmbar ist, wobei der Bildgebungstisch (24) eine Bildgebungsfläche (24A) aufweist, auf die durch die Brust transmittierte Strahlung einfällt, und wobei das Schutzelement (37A) so konfiguriert ist, dass es die Bildgebungsfläche (24A) vor einer Punktionskanüle (43) zum Probennehmen von Gewebe aus der Brust schützt; und
ein Streustrahlenentfernungsraster (44), das Streustrahlen, die von der durch die Brust transmittierenden Strahlung erzeugt werden, entfernt,
wobei das Streustrahlenentfernungsraster (44) ein Raster ist, bei dem mehrere Durchlassteile (47), die die Strahlung transmittieren, und mehrere Absorptionsteile (46), die die Strahlung absorbieren, abwechselnd angeordnet sind und eine Grenzlinie (48) zwischen dem Durchlassteil (47) und dem Absorptionsteil (46) sich in einer Richtung erstreckt,
**dadurch gekennzeichnet, dass**:
das Streustrahlenentfernungsraster (44) mit dem Schutzelement (37A) integriert ist oder abnehmbar an diesem angebracht ist und, wenn das Schutzelement so an dem Bildgebungstisch angebracht ist, dass das Streustrahlenentfernungsraster an einer zugewandten Position, die der Bildgebungsfläche (24A) zugewandt ist, angeordnet ist, das Streuentfernungsraster (44) in einer Stellung ist, in der eine Richtung, in der sich die Grenzlinie (48) erstreckt, senkrecht zu der Richtung ist, die eine Brustwandseite, auf der sich eine Brustwand der Untersuchungsperson befindet, und eine der Brustwand gegenüberliegende Seite verbindet.

2. Schutzabdeckung (37) nach Anspruch 1,
wobei das Streustrahlenentfernungsraster (44) zwischen dem Schutzelement (37A) und der Bildgebungsfläche (24A) angeordnet ist.

3. Schutzabdeckung nach Anspruch 1 oder 2, wobei das Streustrahlenentfernungsraster (44) an dem Schutzelement (37A) in einer nicht anbringbaren/abnehmbaren Form befestigt ist.

4. Schutzabdeckung (37) nach einem der Ansprüche 1 bis 3,
wobei das Schutzelement (37A) mit einer Tragwelle (49) versehen ist, die das Streustrahlenentfernungsraster (44) trägt, und
das Streustrahlenentfernungsraster (44) um die Tragwelle (49) drehbar ist.

5. Schutzabdeckung (37) nach Anspruch 4,
wobei das Schutzelement (37A) eine rechteckige Form aufweist und die Tragwelle (49) an einem von vier Ecken des Schutzelements (37A) angeordnet ist.

6. Schutzabdeckung (37) nach Anspruch 1,
wobei das Schutzelement (37A) mit einem Führungsteil (55) versehen ist, der das Streustrahlenentfernungsraster (44) durch Eingriff mit dem Streustrahlenentfernungsraster (44) zu der zugewandten Position führt.

7. Schutzabdeckung (37) nach Anspruch 6,
wobei der Führungsteil (55) in der Lage ist, das Streustrahlenentfernungsraster (44) von einer Seite, die der Brustwandseite, auf der sich die Brustwand der Untersuchungsperson befindet, gegenüberliegt, aufzunehmen.

8. Schutzabdeckung (37) nach Anspruch 7,
wobei in einem Fall, in dem eine Richtung des Streustrahlenentfernungsrasters (44) entlang der ersten Richtung als eine Tiefenrichtung des Schutzelements (37A) definiert ist und eine Richtung des Streustrahlenentfernungsrasters (44) entlang der zweiten Richtung als eine Breitenrichtung des Schutzelements (37A) definiert ist,
der Führungsteil (55) ein Paar Führungsschienen sind, die an beiden Enden in der Breitenrichtung vorgesehen sind und sich in der Tiefenrichtung erstrecken.

9. Schutzabdeckung (37) nach Anspruch 6,
wobei in einem Fall, in dem eine Richtung des Streustrahlenentfernungsrasters (44) entlang der ersten Richtung als eine Tiefenrichtung des Schutzelements (37A) definiert ist und eine Richtung des Streustrahlenentfernungsrasters (44) entlang der zweiten Richtung als eine Breitenrichtung des Schutzelements (37A) definiert ist,
der Führungsteil (55) in der Lage ist, das Streustrahlenentfernungsraster (44) von einem Endabschnitt des Schutzelements (37A) in der Breitenrichtung aufzunehmen.

10. Schutzabdeckung (37) nach Anspruch 9,
wobei der Führungsteil (55) nur auf einer Seite, die der Brustwandseite in der Tiefenrichtung gegenüberliegt, vorgesehen ist.

11. Schutzabdeckung (37) nach einem der Ansprüche 5 bis 10,
wobei ein Positionierungsteil (37B) vorgesehen ist, der das Streustrahlenentfernungsraster (44), das an dem Schutzelement (37A) angebracht ist, an der zugewandten Position, die der Bildgebungsfläche (24A) zugewandt ist, regelt.

12. Schutzabdeckung (37) nach einem der Ansprüche 5 bis 11,
wobei das Streustrahlenentfernungsraster (44) mit einem Griffteil (44C) versehen ist.

13. Schutzabdeckung (37) nach Anspruch 1,
wobei das Streustrahlenentfernungsraster (44) an dem Schutzelement (37A) so vorgesehen ist, dass es zwischen der zugewandten Position, die der Bildgebungsfläche (24A) zugewandt ist, und einer Rückzugsposition, die von der zugewandten Position zurückgezogen ist, verschoben werden kann; und
wobei das Schutzelement (37A) einen Positionierungsteil (37B) enthält, der das Streustrahlenentfernungsraster (44) verhindert, an der zugewandten Position ersetzt zu werden.

14. Biopsieeinheit (39), umfassend:
die Schutzabdeckung (37) nach einem der Ansprüche 1 bis 13, wobei die Biopsieeinheit (39) die Punktionskanüle (43) beweglich hält.

## Revendications

1. Capot de protection (37) comprenant :
un élément de protection (37A) plat en forme de plaque qui est prévu pour être attaché et détaché d'un appareil de mammographie (10) incluant une table d'imagerie (24) sur laquelle est placé un sein d'un sujet, dans lequel la table d'imagerie (24) comporte une surface d'imagerie (24A) sur laquelle est incident un rayonnement transmis à travers le sein, et dans lequel l'élément de protection (37A) est configuré pour protéger la surface d'imagerie (24A) contre une aiguille de ponction (43) destinée à prélever un échantillon de tissu du sein ; et
une grille d'élimination des rayons diffusés (44) qui élimine des rayons diffusés générés par le rayonnement ayant traversé le sein,
dans lequel la grille d'élimination des rayons diffusés (44) est une grille dans laquelle une pluralité de parties de transmission (47) qui transmettent le rayonnement et une pluralité de parties d'absorption (46) qui absorbent le rayonnement sont disposées en alternance, et une ligne de frontière (48) entre la partie de transmission (47) et la partie d'absorption (46) s'étend dans une direction,
**caractérisé en ce que** :
la grille d'élimination des rayons diffusés (44) est intégrée ou attachée de manière amovible à l'élément de protection (37A), et lorsque l'élément de protection est attaché à la table d'imagerie de sorte que la grille d'élimination des rayons diffusés soit disposée dans une position en regard qui fait face à la surface d'imagerie (24A), la grille d'élimination diffusée (44) est dans une posture dans laquelle une direction dans laquelle s'étend la ligne de frontière (48) est orthogonale à la direction reliant un côté de paroi thoracique, sur lequel se trouve une paroi thoracique du sujet, et un côté opposé à la paroi thoracique.

2. Capot de protection (37) selon la revendication 1,
dans lequel la grille d'élimination des rayons diffusés (44) est disposée entre l'élément de protection (37A) et la surface d'imagerie (24A).

3. Capot de protection selon la revendication 1 ou la revendication 2, dans lequel la grille d'élimination des rayons diffusés (44) est fixée à l'élément de protection (37A) de manière non amovible.

4. Capot de protection (37) selon l'une quelconque des revendications 1 à 3,
dans lequel l'élément de protection (37A) est pourvu d'un arbre de support (49) qui supporte la grille d'élimination des rayons diffusés (44), et
la grille d'élimination des rayons diffusés (44) est rotative autour de l'arbre de support (49).

5. Capot de protection (37) selon la revendication 4,
dans lequel l'élément de protection (37A) a une forme rectangulaire, et l'arbre de support (49) est disposé à l'un quelconque des quatre coins de l'élément de protection (37A).

6. Capot de protection (37) selon la revendication 1,
dans lequel l'élément de protection (37A) est pourvu d'une partie de guidage (55) qui guide la grille d'élimination des rayons diffusés (44) vers la position en regard par engagement avec la grille d'élimination des rayons diffusés (44).

7. Capot de protection (37) selon la revendication 6,
dans lequel la partie de guidage (55) est capable d'accepter la grille d'élimination des rayons diffusés (44) depuis un côté opposé au côté de paroi thoracique sur lequel se trouve la paroi thoracique du sujet.

8. Capot de protection (37) selon la revendication 7,
dans lequel, dans un cas où une direction de la grille d'élimination des rayons diffusés (44) le long de la première direction est définie comme une direction de profondeur de l'élément de protection (37A) et une direction de la grille d'élimination des rayons diffusés (44) le long de la deuxième direction est définie comme une direction de largeur de l'élément de protection (37A),
la partie de guidage (55) est une paire de rails de guidage qui sont prévus aux deux extrémités dans la direction de largeur et s'étendent dans la direction de profondeur.

9. Capot de protection (37) selon la revendication 6,
dans lequel, dans un cas où une direction de la grille d'élimination des rayons diffusés (44) le long de la première direction est définie comme une direction de profondeur de l'élément de protection (37A) et une direction de la grille d'élimination des rayons diffusés (44) le long de la deuxième direction est définie comme une direction de largeur de l'élément de protection (37A),
la partie de guidage (55) est capable d'accepter la grille d'élimination des rayons diffusés (44) depuis une partie d'extrémité de l'élément de protection (37A) dans la direction de largeur.

10. Capot de protection (37) selon la revendication 9,
dans lequel la partie de guidage (55) est prévue uniquement sur un côté opposé au côté de paroi thoracique dans la direction de profondeur.

11. Capot de protection (37) selon l'une quelconque des revendications 5 à 10,
dans lequel une partie de positionnement (37B) est prévue qui régule la grille d'élimination des rayons diffusés (44) attachée à l'élément de protection (37A) à la position en regard qui fait face à la surface d'imagerie (24A).

12. Capot de protection (37) selon l'une quelconque des revendications 5 à 11,
dans lequel la grille d'élimination des rayons diffusés (44) est pourvue d'une partie de préhension (44C).

13. Capot de protection (37) selon la revendication 1,
dans lequel la grille d'élimination des rayons diffusés (44) est prévue sur l'élément de protection (37A) de manière à pouvoir être déplacée entre la position en regard qui fait face à la surface d'imagerie (24A) et une position de retrait qui s'éloigne de la position en regard ; et
dans lequel l'élément de protection (37A) inclut une partie de positionnement (37B) qui empêche que la grille d'élimination des rayons diffusés (44) soit déplacée depuis la position en regard.

14. Unité de biopsie (39) comprenant :
le capot de protection (37) selon l'une quelconque des revendications 1 à 13, l'unité de biopsie (39) maintenant de manière mobile l'aiguille de ponction (43).
